# EUROPEAN PATENT APPLICATION

(11) **EP 2 264 027 A1**
(43) Date of publication of application: **22.12.2010**
(21) Application number: 09007080.6
(22) Date of filing: 27.05.2009
(51) Int. Cl.: C07D 403/06

(54) **Process for the preparation of N-[2-(Diethylamino)ethyl]-5-[(5-fluoro-1,2-dihydro-2-oxo-3H-indol-3-ylidene) methyl]-2,4-dimethyl-1H-pyrrole-3-carboxamide**

(71) Applicant: Ratiopharm GmbH, 89079 Ulm (DE)
(72) Inventor: Christ, Jochen, 89081 Ulm (DE); Striegel, Hans-Günter, 89143 Blaubeuren (DE)
(74) Representative: Teipel, Stephan

(57) **Abstract**

The present invention relates to processes for the preparation of N-[2-(Diethylamino)ethyl]-5-[(5-fluoro-1,2-dihydro-2-oxo-3H-indol-3-ylidene)methyl]-2,4-dimethyl-1H-pyrrole-3-carbox- amide of formula (VI).

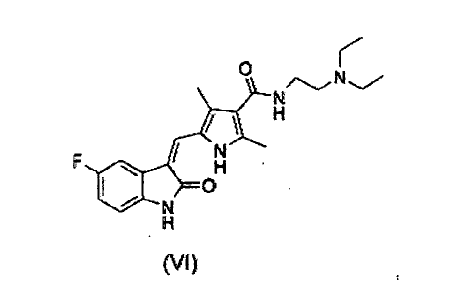

## Description

The present invention relates to processes for the preparation of N-[2-(Diethylamino)ethyl]-5-[(5-fluoro-1,2-dihydro-2-oxo-3H-indol-3-ylidene)methyl]-2,4-dimethyl-1H-pyrrole-3-carboxamide.

The compound N-[2-(diethylamino)ethyl]-5-[(5-fluoro-1,2-dihydro-2-oxo-3H-indol-3-ylidene)methyl]-2,4-dimethyl-1H-pyrrole-3-carboxamide (also known and referred to herein as Sunitinib) has the following chemical structure: 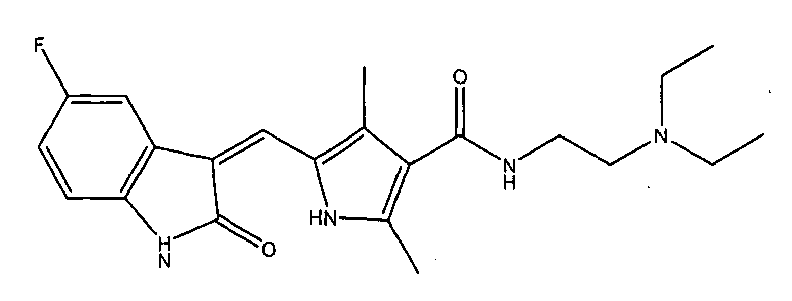 and is a receptor tyrosine kinase inhibitor which is used to treat disorders like renal cell carcinoma (RCC) and gastrointestinal stromal tumors (GIST). Its activity relies on the inhibition of cellular signaling by targeting multiple receptor tyrosine kinases including platelet-derived growth factor receptors and vascular endothelial growth factor receptors. Since both kinds of receptors are involved in tumor angiogenesis and tumor cell proliferation, the simultaneous inhibition of these targets results in both reduced tumor vascularization and cancer cell death. These effects are responsible for the finally observed shrinkage of the renal cell carcinoma and gastrointestinal stromal tumors, respectively.

Sunitinib and its pharmaceutical effects on disorders like cancer are described in WO 01/60814. Further medical uses of Sunitinib and its salts are inter alia known from WO 01/45689 and WO 03/035009.

Typically, Sunitinib is administered in a dose of 50 mg once daily, which, if necessary, has to be varied according to individual tolerance and safety. Thus, in order to obtain sufficiently flexible dosing, individual dosage forms generally contain 12.5, 25 and 50 mg Sunitinib. Capsules comprising the malate salt of Sunitinib are sold under the brand name Sutent® (by Pfizer Pharma).

A number of processes for the synthesis of Sunitinib are known. WO 01/60814 discloses two processes for preparing Sunitinib.

US 2006/0009510 A1 discloses a process for the preparation of inter alia Sunitinib according to the following scheme 1:

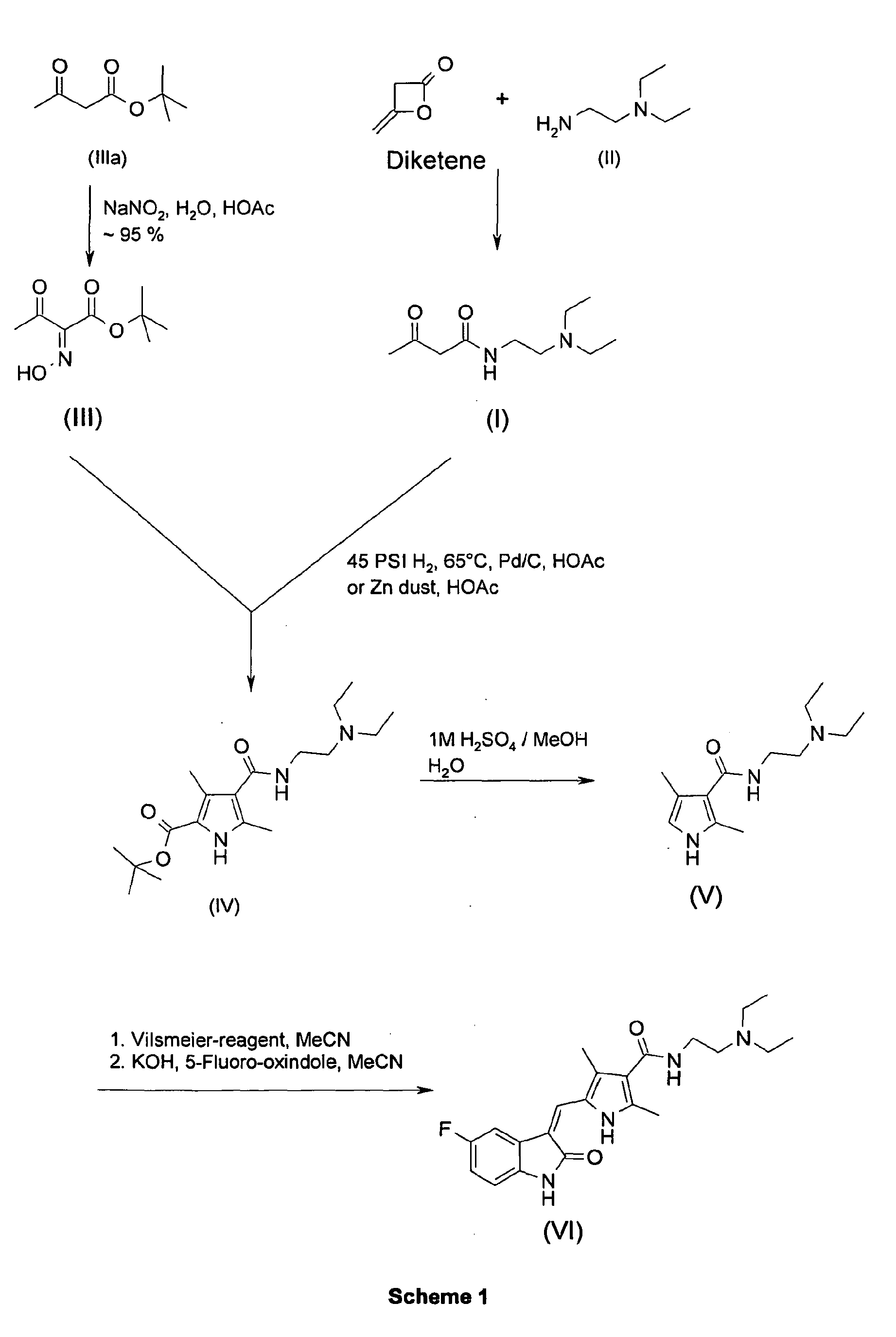

According to scheme 1, the compound of the formula I is obtained by reacting the compound of formula II with diketene (γ-methylene butyrolactone). However, application of diketene is disadvantageous because of its reactivity and toxicity. Further handling with unstabilized diketene is legally regulated in some countries, which renders the application of the substance disadvantageous, in particular for industrial application.

Therefore, there is still a need for a further method of synthesizing the compound according to formula I, in particular as intermediate for the preparation of compounds of the formula VI, e.g. Sunitinib.

It has now been found that the compound of the formula II can also be reacted with the diketene-acetone adduct 2,2,6-trimethyl-1,3-dioxin-4-one to obtain the compound of formula I (cf. scheme 2).

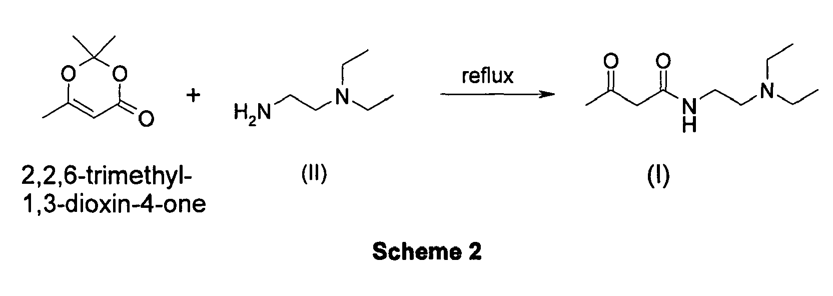

The advantage of this reaction is that no diketene has to be used and the product, i.e. the compound of formula I, is obtained in good yields and purity, such that it can be directly applied for further reactions, in particular in a process for the preparation of a compound of formula IV, V, and/or VI, e.g. Sunitinib.

Therefore, the present invention relates to a process for the preparation of a compound of the formula I 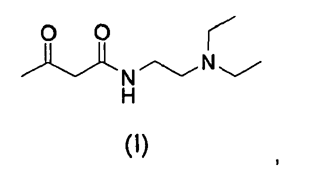 or a salt thereof, which process comprises the step of reacting a compound of the formula II 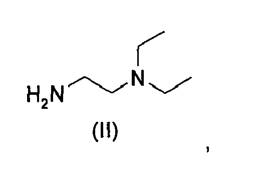 or a salt thereof, with 2,2,6-trimethyl-1,3-dioxin-4-one.

The compound of the formula I prepared by the process of the present invention is intended as intermediate for the preparation of a compound of the formulas IV, V and/or VI, in particular Sunitinib in its polymorphic form II or III or a salt thereof, e.g. the L-(-)-malate salt.

The reaction of the compound of the formula II with 2,2,6-trimethyl-1,3-dioxin-4-one is preferably conducted under reflux in an appropriate solvent or without solvent, e.g. at a temperature of about 110 to about 115°C. The generated acetone can be distilled off during the reaction. The obtained compound of the formula I can be purified as known in the art, but is preferably further reacted as crude product, which typically contains some unreacted amine as well as some traces of acetone, in the reaction with a compound of the formula III.

Preferably, the compound of the formula I 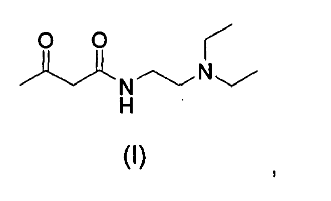 or a salt thereof, is further reacted with a compound of the formula III 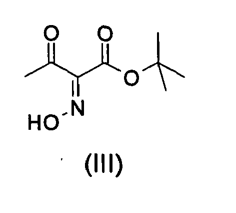 to obtain a compound of the formula IV 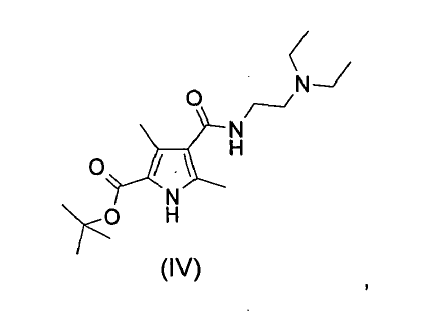 or a salt thereof. The compound of the formula IV is also intended as intermediate for the preparation of compounds if the formulae V or VI, in particular Sunitinib or a salt thereof.

The compound of the formula III can be obtained as described in WO 2001/37820 or US 2006/0009510 A1. Thus, the compound of the formula III is preferably obtained by nitrosation for a compound of the formula IIIa, e.g. by applying an aqueous solution of sodium nitrite in acetic acid (cf. scheme 3).

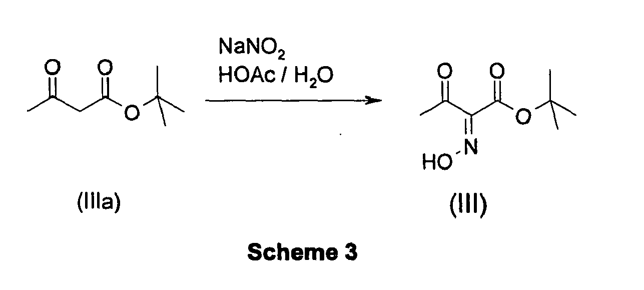

The reaction of the compound of the formula I with a compound of the formula III can be conducted as described in US 2006/0009510 A1. Thus, the compound of the formula I and III are preferably reacted under hydrogenating conditions, e.g. under an increased pressure of hydrogen, e.g. about 5 bar, in a suitable solvent, such as acetic acid. A suitable hydrogenation catalyst, such as palladium on activated charcoal, is preferably applied. The reaction of the compound of the formula I with the compound of the formula III is preferably conducted as illustrated in the following scheme 4:

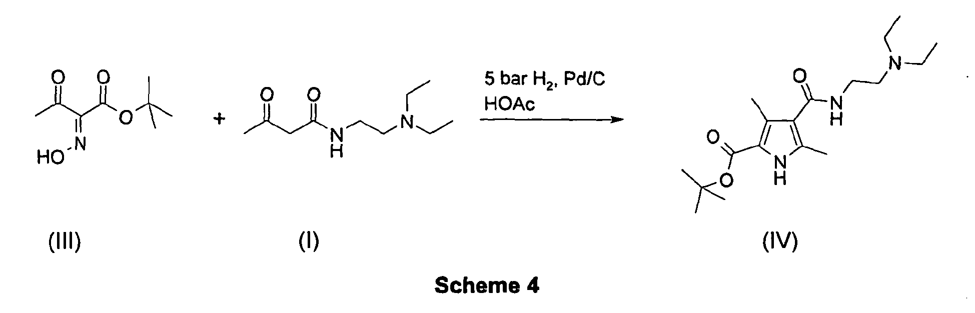

Alternatively, the reaction according to scheme 4 can be conducted under a pressure of hydrogen of about 3 bar, more preferably in combination with a pre-warming of the solvent to about 65°C. Thereby, high yields of the product, i.e. the compound of the formula IV, can be obtained with excellent purities of above 99 %.

Preferably, according to the present invention, the compound of the formula IV 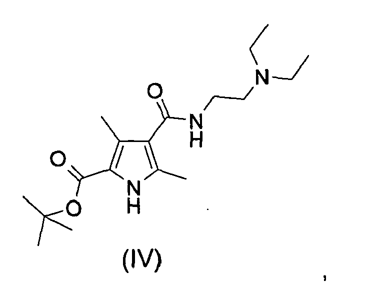 or a salt thereof, is further reacted to obtain a compound of the formula V 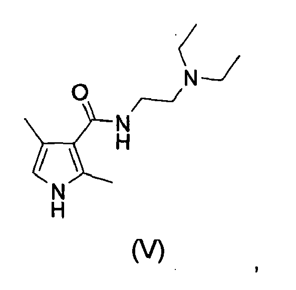 or a salt thereof.

This reaction can be conducted as described in US 2006/0009510 A1, i.e. the saponification and decarboxilation of the compound of the formula IV is conducted by application of a solution of about 1 M sulphuric acid in methanol:water in a ratio of about 3:1.

However, in particular for industrial application of this process it is desirable to reduce the amount of organic waste, in this case of the methanol solvent.

It has surprisingly been found that the amount of organic solvent, i.e. methanol, can be reduced or even avoided without reduction of the yields obtained. Thus, the process of the present invention preferably comprises the step of reacting the compound of the formula IV to obtain a compound of the formula V, which process is conducted without applying organic solvent, more preferably an aqueous solution of sulfuric acid of about 1 M is applied in the step, i.e. this step is most preferably conducted according to the following scheme 5:

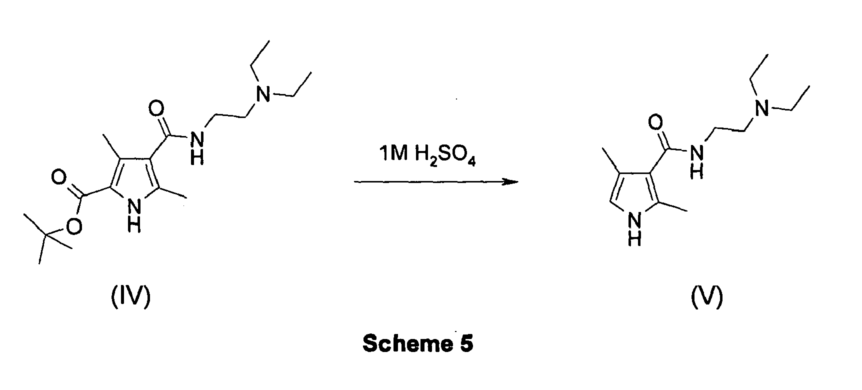

The reaction according to scheme 5 has the additional advantage that without application of an organic solvent in the reaction, such as methanol, a better phase separation is achieved during work up of the product, which is advantageous, in particular for industrial application.

Preferably, according to the present invention, the compound of the formula V, or a salt thereof, is further reacted to obtain a compound of the formula VI 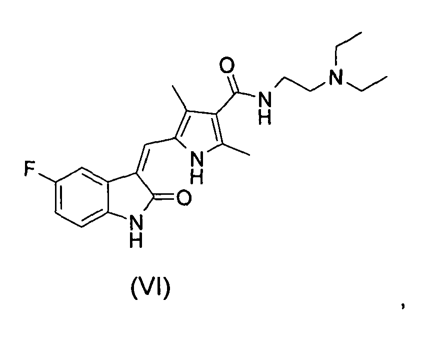 or a salt thereof.

The reaction of the compound of the formula V to obtain a compound of the formula VI can be conducted as described in US 2006/0009510 A1, i.e. the compound of the formula V is first formylated by means of the Vilsmeier reagent, which reagent is commercially available, and then condensed with 5-fluoro-oxindole. The obtained compound of the formula VI is typically obtained in the free base form, i.e. Sunitinib in its free base form. The obtained Sunitinib is, as far as it is crystalline, typically obtained in the crystalline form II (crystalline Sunitinib in the polymorphic form II).

Alternatively, the reaction of the compound of the formula V to obtain the compound of the formula VI can be carried out with in situ generation of the Vilsmeier reagent. Preferably, the Vilsmeier reagent is generated in situ by adding a solution of oxalyl chloride (COCl₂), in an appropriate solvent, such as acetonitrile, to a solution of dimethyl formamide (DMF), optionally in an appropriate solvent, such as acetonitrile, and preferably followed by mixing for an appropriate time, such as about 40 minutes to about 1 hour. Thus, the reaction of the compound of formula V to obtain a compound of the formula VI is most preferably conducted according to the following scheme 6:

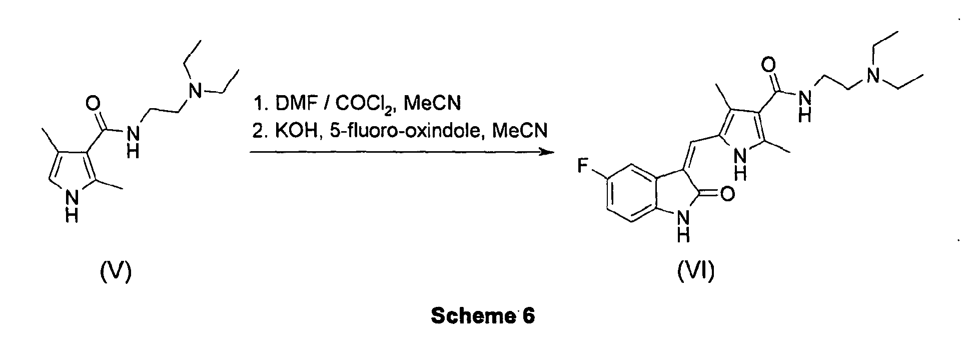

Application of an in situ generated Vilsmeier reagent leads to high yields of about 60 % of Sunitinib free base with chemical purities of about 96 to about 99 %.

As further alternative, the oxalyl chloride can be added to a solution of the compound of the formula IV in presence of dimethyl formamide in the first step and addition of the 5-fluoro-oxindole and a suitable base, such as potassium hydroxide, in the second step. In this alternative, Sunitinib free base can be obtained in an acceptable yield and high chemical purity of about 95 %.

From the above described process the compound of the formula VI is typically obtained in its free base form, i.e. Sunitinib in its free base form. If this Sunitinib in its free base form is crystallized, e.g. during the work-up, typically the Sunitinib is obtained in the crystalline polymorphic form II. Preferably the Sunitinib in polymorphic form II is, in a further step, recrystallized to obtain crystalline Sunitinib in its polymorphic form III, or alternatively or subsequently to recrystallization to polymorphic form III (i.e. in an alternative or subsequent process step), reacted to obtain the L-(-)-malate salt of the compound of the formula VI, i.e. L-(-)-malate salt of Sunitinib.

The term "crystalline" refers to any substantially, preferably completely non amorphous forms of the active pharmaceutical ingredient. The term "amorphous form" refers to a form of active pharmaceutical ingredient which has no long-range order like crystalline forms. The atoms or molecules of a material present in amorphous form are arranged in a non-uniform array. It is for example possible to distinguish amorphous from crystalline forms of a compound by powder X-ray diffraction.

### Abbreviations

- A: Angstrom
- DSC: differential scanning calorimetry
- DMSG: dynamic moisture sorption gravimetry
- IR: infrared
- RH: relative humidity
- RT: room temperature
- SCXRD: singe crystal X-ray diffraction
- XRPD: X-ray powder diffraction

### Brief description of the figures

Figure 1: XRPD pattern of Sunitinib in the polymorphic form I
Figure 2: XRPD pattern of Sunitinib in the polymorphic form II
Figure 3: XRPD pattern of Sunitinib in the polymorphic form III

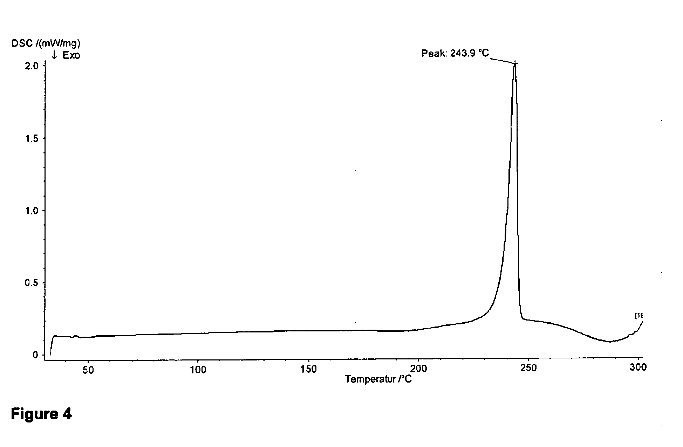

Figure 4: DSC thermogram of Sunitinib in the polymorphic form I

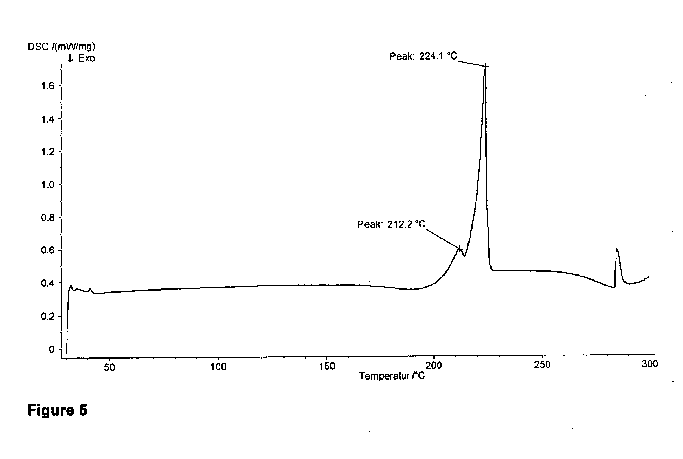

Figure 5: DSC thermogram of Sunitinib in the polymorphic form II

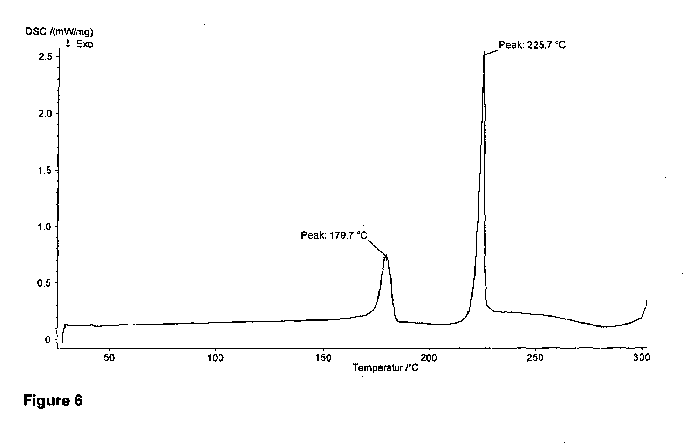

Figure 6: DSC thermogram of Sunitinib in the polymorphic form III

### Polymorph I of Sunitinib

The present invention describes a yellow to greenish yellow solid that will in the following be referred to as polymorph I of Sunitinib. It is characterized by an XRPD pattern having a characteristic peak at 4.5 ± 0.2 degrees 2-theta, in particular with peaks at 4.5 ± 0.2; 9.1 ± 0.2; 16.8 ± 0.2 and 26.0 ± 0.2 degrees 2-theta.

Furthermore, polymorph I of Sunitinib can be characterized by an XRPD pattern with peaks at 4.5 ± 0.2; 9.1 ± 0.2; 15.2 ± 0.2; 16.8 ± 0.2; 18.3 ± 0.2; 20.4 ± 0.2; 21.8 ± 0.2; 22.9 ± 0.2 and 26.0 ± 0.2.degrees 2-theta.

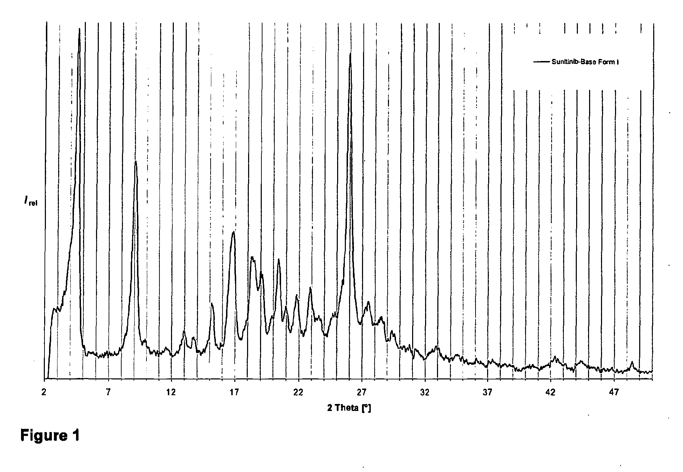

The XRPD pattern of polymorph I of Sunitinib is shown in figure 1.

Polymorph I of Sunitinib shows a Raman spectrum exhibiting characteristic peaks at 2927 ± 2 cm⁻¹, 1679 ± 2 cm⁻¹, 1585 ± 2 cm⁻¹, 1437 ± 2 cm⁻¹, 1334 ± 2 cm⁻¹, 1278 ± 2 cm⁻¹ and 670 ± 2 cm⁻¹.

Polymorph I of Sunitinib shows an IR spectrum exhibiting characteristic peaks at 2969 ± 2 cm⁻¹, 1479 ± 2 cm⁻¹, 1330 ± 2 cm⁻¹, 1189 ± 2 cm⁻¹, 797 ± 2 cm⁻¹, 667 ± 2 cm⁻¹ and 609 ± 2 cm⁻¹.

Polymorph I of Sunitinib exhibits a low hygroscopicity. DMSG revealed a moisture sorption of maximum about 1 % referred to the weight of the sample.

### Polymorph II of Sunitinib

The present invention further describes polymorph II of Sunitinib. This crystal form has the following characteristics:
- crystal system triclinic
- space group P-1
- cell metrics
   a = 13.5 ± 0.2 Å
   b = 14.4 ± 0.2 Å
   c = 24.3 ± 0.2 Å
   α = 79 ± 1 °
   β = 81 ± 1 °
   γ = 89 ± 1 °
- cell volume V 4598.85 Å³
- molecules per unit cell 8

Polymorph II of Sunitinib is characterized by an XRPD pattern having a characteristic peak at 3.8 ± 0.2 degrees of 2-theta, in particular with peaks at 3.8 ± 0.2; 9.0 ± 0.2; 14.0 ± 0.2; 18.1 ± 0.2 and 20.5 ± 0.2 degrees 2-theta.

Furthermore, polymorph II of Sunitinib can be characterized by an XRPD pattern with peaks at 3.8 ± 0.2; 9.0 ± 0.2; 14.0 ± 0.2; 18.1 ± 0.2; 20.5 ± 0.2; 26.6 ± 0.5 and 27.5 ± 0.6 degrees 2-theta.

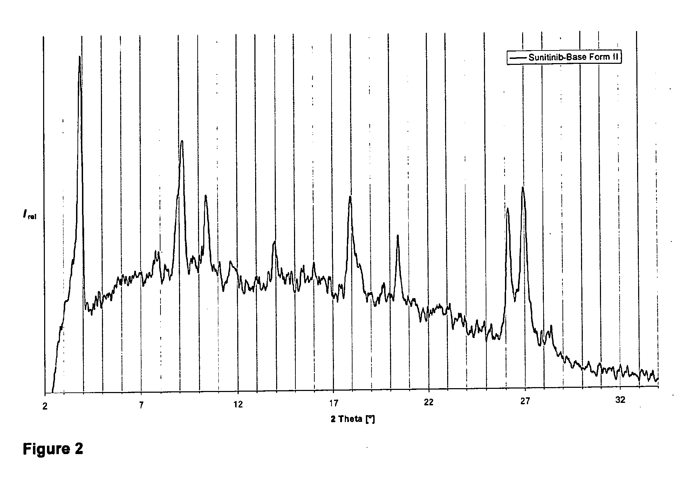

The XRPD of polymorph II of Sunitinib is shown in figure 2.

Polymorph II of Sunitinib shows a Raman spectrum exhibiting characteristic peaks at 2929 ± 2 cm⁻¹, 1627 ± 2 cm⁻¹, 1583 ± 2 cm⁻¹, 1425 ± 2 cm⁻¹, 1328 ± 2 cm⁻¹, 1285 ± 2 cm⁻¹, 1264 ± 2 cm⁻¹ and 669 ± 2 cm⁻¹.

Polymorph II of Sunitinib shows an IR spectrum exhibiting characteristic peaks at 1667 ± 2 cm⁻¹, 1476 ± 2 cm⁻¹, 1325 ± 2 cm⁻¹, 1147 ± 2 cm⁻¹, 794 ± 2 cm⁻¹, 668 ± 2 cm⁻¹ and 608 ± 2 cm⁻¹.

Sunitinib in the polymorphic form II exhibits some hygroscopicity. DMSG revealed a moisture sorption of more than 6 % referred to the weight of the sample.

Polymorph II of Sunitinib is obtainable by a process, comprising the steps of:
- dissolving Sunitinib present in any polymorphic or amorphous form or mixtures thereof, preferably in its polymorphic form I in a suitable inert solvent, preferably in water or an organic solvent or a mixture of two or more suitable inert solvents, preferably at a temperature between 25 °C and the reflux temperature of the solvent or the solvent mixture,
- optionally adding another solvent, preferably another organic solvent,
- concentrating the solution, preferably under reduced pressure, more preferably at a temperature between 25 °C and the reflux temperature of the solvent or the solvent mixture, most preferably under reduced pressure at 25 to 60 °C,
- collecting the resulting crystalline precipitate, preferably by filtration.

The crystallization is carried out in a suitable inert solvent or in a mixture of two or more suitable inert solvents. Examples of such suitable inert solvents are water, aliphatic and aromatic hydrocarbons (preferably hexane, benzene, toluene or xylene), aliphatic alcohols (preferably methanol, ethanol, propanol, iso-propanol), ethers (preferably diethyl ether, diisopropyl ether or dimethoxyethane), cyclic ethers (preferably tetrahydrofuran or dioxane), ketones (preferably acetone, methylisobutylketone or methylethylketone), esters (preferably ethylacetate), chlorinated hydrocharbons (preferably dichloromethane or chloroform) or nitrogen containing organic solvents (preferably N-methyl pyrollidone, dimethylformamide or acetonitrile). Acetone and toluene are especially preferred.

An alternative process for the preparation of polymorph II of Sunitinib comprises the steps of:
- dissolving a salt of Sunitinib, preferably a salt of Sunitinib as obtained by the above described process of the present invention (or as disclosed in WO 01/060814), more preferably the malate salt, in a suitable inert solvent, preferably in water, or a mixture of two or more suitable inert solvents optionally heating the solvent to a temperature between 25 °C and the reflux temperature of the solvent or the solvent mixture,
- adding a base, preferably NaOH,
- collecting the resulting crystalline precipitate, preferably by filtration.

As solvents the suitable inert solvents as described above can be used.

The above described reaction is carried out in the presence of a base, preferably a Bronsted base. Examples of suitable Bronsted bases are metal hydroxides, metal carbonates or amines, preferably alkali metal hydroxides, alkali metal carbonates, alkali metal bicarbonates, ammonia, or organic amines, such as, for example, sodium hydroxide, potassium hydroxide, sodium carbonate, potassium carbonate, sodium bicarbonate, ammonia, diethylamine, triethylamine, diisopropylamine or pyridine. Sodium hydroxide is preferred. The base is used in an amount sufficient to obtain the free base of Sunitinib from its salt.

### Polymorph III of Sunitinib

The present invention further describes polymorph III of Sunitinib. This crystal form has the following characteristics:
- crystal system monoclinic
- space group P 2₁/n
- cell metrics
   a = 4.97560(10) Å
   b = 28.1365(6) Å
   c = 14.5880(3) Å
   β = 93.5130(10) °
- cell volume V 2038.42(7) Å³
- molecules per unit cell 4

Polymorph III of Sunitinib is characterized by an XRPD pattern having a characteristic peak at 6.3 ± 0.2 degrees 2-theta, in particular with peaks at 6.3 ± 0.2; 22.2 ± 0.2 and 26.4 ± 0.2 degrees 2-theta.

Furthermore, polymorph III of Sunitinib can be characterized by an XRPD pattern with peaks at 6.3 ± 0.2; 14.0 ± 0.2; 15.4 ± 0.2, 18.9 ± 0.2, 19.3 ± 0.2; 22.2 ± 0.2; 24.2 ± 0.2 and 26.4 ± 0.2 degrees 2-theta.

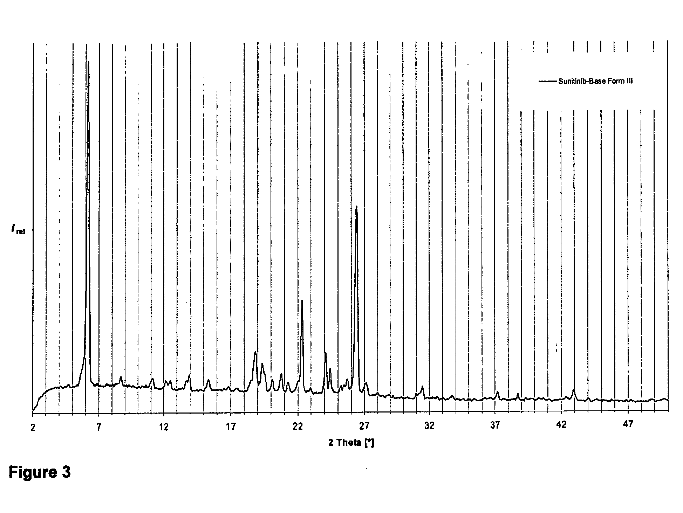

The XRPD pattern of polymorph III of Sunitinib is shown in figure 3.

Polymorph III of Sunitinib shows a Raman spectrum exhibiting characteristic peaks at 1674 ± 2 cm⁻¹, 1569 ± 2 cm⁻¹, 1414 ± 2 cm⁻¹, 1327 ± 2 cm⁻¹, 1297 ± 2 cm⁻¹, 1259 ± 2 cm⁻¹, 1030 ± 2 cm⁻¹ and 666 ± 2 cm⁻¹.

Polymorph III of Sunitinib shows an IR spectrum exhibiting characteristic peaks at 3435 ± 2 cm⁻¹, 1670 ± 2 cm⁻¹, 1473 ± 2 cm⁻¹, 1294 ± 2 cm⁻¹, 1194 ± 2 cm⁻¹, 1146 ± 2 cm⁻¹, 786 ± 2 cm⁻¹, 663 ± 2 cm⁻¹, 617 ± 2 cm⁻¹.

Polymorph III exhibits a very low hygroscopicity. Under DMSG, the sample does not gain weight, even at a relative humidity of 95 %.

Polymorph III is preferably further characterized by the absence of any solvent molecules within the crystal.

A process for the preparation of polymorph III of Sunitinib comprises the steps of:
- preparing a clear saturated solution of Sunitinib, preferably by the above described process of the present invention, in a suitable inert solvent,
- cooling the solution and allowing Sunitinib to crystallize,
- removing the resulting precipitate,
- keeping the filtrate at room temperature until Sunitinib crystallizes in the form of dark orange needles
or alternatively:
- preparing a clear saturated solution of Sunitinib, preferably by the above described process of the present invention, in a suitable inert solvent,
- cooling the solution and seeding it with crystals of polymorph III of Sunitinib,
- allowing polymorph III to crystallize from the seeded solution at room temperature in the form of dark orange needles.

Preferably the process comprises the steps of:
- preparing a suspension of Sunitinib by suspending Sunitinib present in any polymorphic or amorphous form or mixtures thereof, preferably in its polymorphic form I or II, in a suitable inert solvent, preferably in an organic solvent or a mixture of two or more organic solvents, which is preferably obtained according to an above described process,
- heating the suspension, preferably to the reflux temperature of the solvent or the solvent mixture, optionally by adding an additional amount of solvent, until a clear and saturated solution is obtained,
- cooling the solution and allowing Sunitinib to precipitate,
- removing the resulting precipitate, preferably by filtration,
- keeping the filtrate at room temperature until Sunitinib crystallizes in the form of dark orange needles,
or alternatively:
- preparing a suspension of Sunitinib by suspending Sunitinib present in any polymorphic or amorphous form or mixtures thereof, preferably in its polymorphic form I or II in a suitable inert solvent, preferably in an organic solvent or a mixture of two or more organic solvents, which is preferably obtained according to an above described process,
- heating the saturated suspension, preferably to the reflux temperature of the solvent or the solvent mixture, optionally by adding an additional amount of solvent, until a clear and saturated solution is obtained,
- cooling the solution and seeding it with crystals of polymorph III of Sunitinib,
- allowing polymorph III of Sunitinib to crystallize from the seeded solution at room temperature in the form of dark orange needles.

The above described crystallizations are carried out in suitable inert solvents or mixtures of two or more suitable inert solvents. Examples of such suitable inert solvents are water, aliphatic and aromatic hydrocarbons (preferably hexane, benzene, toluene or xylene), aliphatic alcohols (preferably methanol, ethanol, propanol, iso-propanol), ethers (preferably diethyl ether, diisopropyl ether or dimethoxyethane), cyclic ethers (preferably tetrahydrofuran or dioxane), ketones (preferably acetone, methylisobutylketone or methylethylketone), esters (preferably ethylacetate), chlorinated hydrocharbons (preferably dichloromethane or chloroform) or nitrogen containing organic solvents (preferably N-methyl pyrollidone, dimethylformamide or acetonitrile). Acetone and toluene are especially preferred.

Preferably, the process according to the present invention further comprises the step of reacting the compound of the formula VI, e.g. in one of the polymorphic forms as described above, or a salt thereof, to obtain Sunitinib-L-(-)-malate, e.g. according to the following scheme 7:

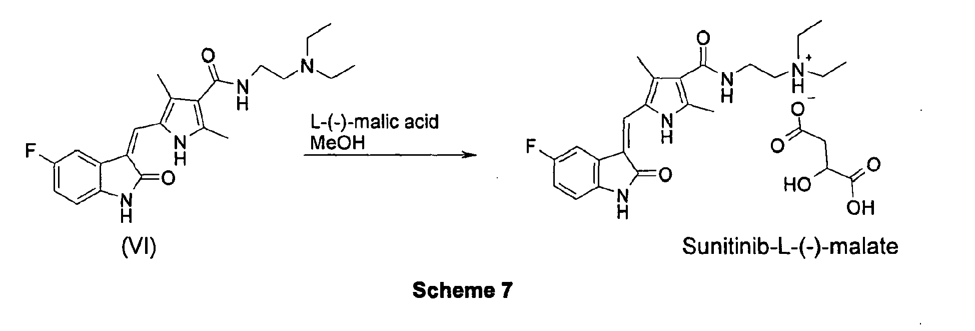

The preparation of the Sunitinib-L-(-)-malate salt from the free base form can be conducted as known in the art, e.g. described in WO 2001/60814. Preferably, an alternative procedure can be applied, wherein the Sunitinib free base is suspended in pre-warmed organic solvent, e.g. methanol, preferably at about 50°C, followed by the addition of L-(-)-malic acid. The product can be obtained by cooling, preferably after dissolution of all solids, and filtering. The latter procedure leads to yields of up to about 90 % of the theoretical yield of Sunitinib L-(-)-malate in excellent chemical purity of >99 %.

The present invention further relates to processes which comprise one or more of the individual process steps as described above, in particular one or more of the process steps illustrated in schemes 2 to 7.

The invention is further illustrated by the following examples, which are not constructed as being limiting.

### Example 1: Synthesis of 2-(Hydroxyimino)-3-oxo-butyric acid tert-butyl ester (compound of the formula III)

The compound of the formula III was synthesized by nitrosation of tert-butyl acetonate (compound of the formula IIIa) with an aqueous solution of sodium nitrite in acetic acid (according to US 2006/0009510 A1).

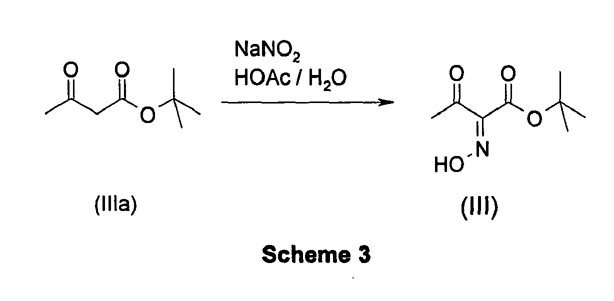

### Experimental procedure

Aqueous sodium nitrite solution (2 equiv.) was added drop wise to an ice-cooled solution of tert-butyl acetoacetate in acetic acid keeping the temperature below 15°C. After the addition, the solution was warmed to room temperature and stirred for 1 h. Then, brine and diethyl ether were added, the phases were separated and the aqueous phase was extracted with further diethyl ether. The combined organic layers were washed twice with water and dried over sodium sulphate. The solvent was evaporated in vacuum yielding about 90 % of the crude product which was used without further purification.

**Table 1: Overview of synthesis of 2-(hydroxyimino)-3-oxo-butyric acid tert-butyl ester (compound of the formula III)**

| Experiment | Starting material (IIIa) | | Product (III) | | | |
|---|---|---|---|---|---|---|
| No. | [mol] | [g] | [g] | Yield (%) | Purity (%) | Remarks and observation |
| 1 | 0.190 | 30.0 | 36.22 | 102.0 | n.d. | residual acetic acid was eliminated by aceotropic distillation with toluene |
| | | | | | | raw material, used without further purification |
| 2 | 1.000 | 158.2 | 186 | 99.4 | 87.0 | raw material, used without further purification |
| 3 | 4.000 | 632.8 | 705 | 94.2 | 83.0 | raw material, used without further purification |
| 4 | 4.000 | 632.8 | 769 | 102.7 | 84.0 | raw material, used without further purification |
| 5 | 4.000 | 632.8 | 710 | 94.8 | 68.0 | raw material, used without further purification |
| 6 | 4.000 | 632.8 | 699 | 93.3 | 84.0 | raw material, used without further purification |
| 7 | 6.000 | 949.2 | 1092 | 97.2 | 88.0 | raw material, used without further purification |

### Example 2: Synthesis of N-(2-Diethylamino-ethyl)-3-oxo-butyramide (compound of the formula I)

Diketene-acetone adduct was reacted with N,N-diethylethylenediamine under reflux conditions to give the compound of the formula I.

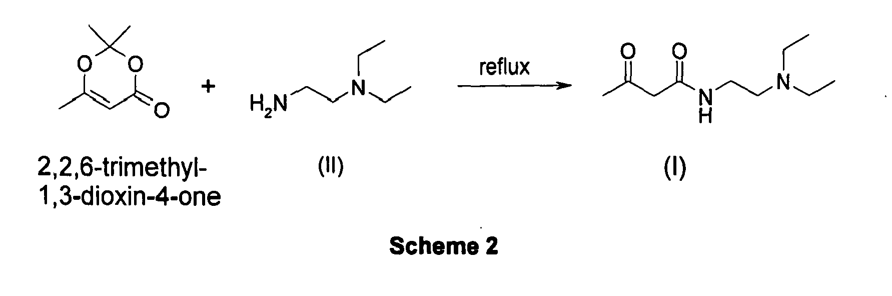

### Experimental procedure

Diketene-acetone adduct (2,2,6-trimethyl-1,3-dioxin-4-one; 1 equiv.) and N,N-diethylethylenediamine (1.1 equiv.) were heated to reflux (oil bath temperature 110-115°C). The generated acetone was distilled off during the reaction. The crude product, still containing some unreacted amine as well as some traces of acetone was directly used in the next step without further purification.

**Table 2: Overview of synthesis of N-(2-Diethylamino-ethyl)-3-oxo-butyramide (compound of the formula I)**

| Experiment | Starting material (2,2,6-trimethyl-1,3-dioxin-4-one) | | Starting material (II) | | Product (I) | | | |
|---|---|---|---|---|---|---|---|---|
| No. | [mol] | [g] | [mol] | [g] | [g] | Yield (%) | Purity (%) | Remarks and observation |
| 8 | 0.038 | 5.45 | 0.040 | 4.61 | 7.07 | 98.0 | n.d. | raw material, used without further purification |
| 9 | 0.10 | 14.22 | 0.10 | 12.02 | 20.5 | 108.9 | 82.0 | raw material, used without further purification |
| 10 | 0.40 | 56.86 | 0.41 | 48.06 | n.d. | n.d. | n.d. | raw material, used without further purification |
| 11 | 0.8 | 113.73 | 0.83 | 96.13 | n.d. | n.d. | n.d. | raw material, used without further purification |
| 12 | 1.6 | 227.46 | 1.65 | 192.26 | n.d. | n.d. | n.d. | raw material, used without further purification |
| 13 | 1.6 | 227.46 | 1.65 | 192.26 | n.d. | n.d. | n.d. | raw material, used without further purification |
| 14 | 2.5 | 355.4 | 2.59 | 300.40 | n.d. | n.d. | n.d. | raw material, used without further purification |
| 15 | 2.5 | 355.4 | 2.59 | 300.40 | n.d. | n.d. | n.d. | raw material, used without further purification |
| 16 | 2.5 | 355.4 | 2.59 | 300.40 | n.d. | n.d. | n.d. | raw material, used without further purification |
| 17 | 2.5 | 355.4 | 2.59 | 300.40 | n.d. | n.d. | n.d. | raw material, used without further purification |
| 18 | 2.5 | 355.4 | 2.59 | 300.40 | n.d. | n.d. | n.d. | raw material, used without further purification |

### Example 3: Synthesis of 3,5-Dimethyl-1H-pyrrole-2,4-dicarboxylic acid 2-tert-butyl ester 4-(2-diethylamino-ethyl)-amide (compound of the formula IV)

2-(Hydroxyimino)-3-oxo-butyric acid tert-butyl ester (referred to herein as "oxime", i.e. compound of the formula III) and N-(2-Diethylamino-ethyl)-3-oxo-butyramide (compound of the formula I) were hydrogenated under increased pressure in acetic acid with palladium on activated charcoal as catalyst.

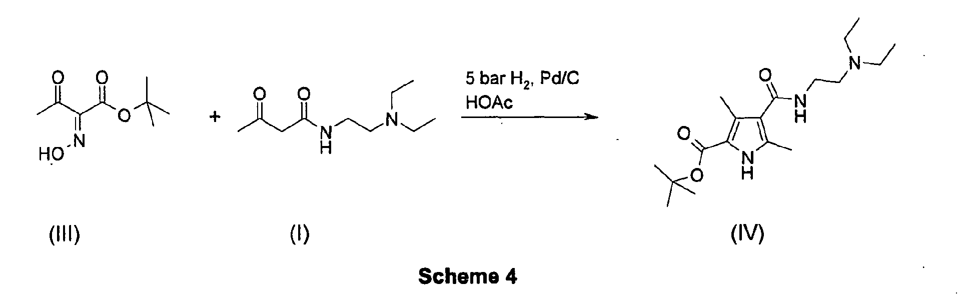

### Experimental procedure

Oxime (compound of the formula III) (0.87 equiv.) and the crude amide (compound of the formula I) (1 equiv.) were independently dissolved in acetic acid and palladium on activated charcoal (0.9 %) was added. After inertising with nitrogen, the mixture was hydrogenated.

The first 5 batches were hydrogenated under normal pressure at room temperature for up to 7 days (the reaction stopped several times and more catalyst had to be added). After the hydrogenation was completed, the catalyst was removed by filtration over kieselgur and the cake was washed with excess acetic acid. The solvent was reduced under vacuum and the residue neutralised with 10 % aqueous sodium hydroxide to pH 10-11. The aqueous phase was extracted several times, either with ethyl acetate or with dichloromethane, the combined organic phases washed twice with water and the washing phases back-extracted with little of the organic solvent. The combined organic layers were dried with sodium sulphate and reduced to dryness. The residue was recrystallized from acetonitrile. This protocol yielded 30-47 % of the desired product in 92-99.9 % purity.

In order to avoid the difficulties adding more catalyst and the long reaction times, the hydrogenation reaction was conducted in an alternative procedure under increased pressure. The reactants were dissolved, added to a hydrogenation vessel and palladium on activated charcoal was added. The mixture was hydrogenated at 5 bar at room temperature, whereby it showed a highly exothermic profile with temperatures up to about 80°C. After the same work-up procedure as described above, constant yields (46-54 %) and purities (> 99 %) were obtained.

As further alternative, hydrogenation was performed under 3 bar of hydrogen, and the mixture was preliminarily warmed to 65°C. These batches resulted, also after the work-up procedure described above, in 43.5 % and 46 % yield respectively, the purity was constantly > 99 %.

**Table 3: Overview of synthesis of 4-(2-Diethylamino-ethylcarbamoyl)-3,5-dimethyl-1H-pyrrole-2-carboxylic acid tert-butyl ester (compound of the formula IV)**

| Experiment | Starting material (III) | | Starting material (I) | | Product (IV) | | | |
|---|---|---|---|---|---|---|---|---|
| No. | [mol] | [g]^{*} | [mol] | [g] | [g] | Yield (%) | Purity (%) | Remarks and observation |
| 19 | 0.035 | 6.55 | 0.035 | 7.01 | 5.47 | 46.3 | 91.6 | hydrogenated at ambient pressure and temperature, extracted with dichloromethane |
| 20 | 0.10 | 22.46 | 0.10 | 19.71 | 15.8 | 47.6 | 98.5 | hydrogenated at ambient pressure and temperature, extracted with dichloromethane |
| 21 | 0.33 | 74.68 | - | - | 60.0 | 47.3^{*} | 99.6 | hydrogenated at ambient pressure and temperature, extracted with dichloromethane |
| 22 | 0.75 | 171.68 | - | - | 116.0 | 45.7^{*} | 99.9 | product of experiment No. 11 was directly used, hydrogenated at ambient pressure and temperature, extracted with dichloromethane |
| 23 | 1.50 | 343.35 | - | - | 151.0 | 29.8^{*} | 99.9 | product of experiment No. 12 was directly used, hydrogenated at ambient pressure and temperature, addition of more catalyst necessary, extracted with dichloromethane |
| 24 | 1.50 | 343.35 | - | - | 253.5 | 49.9^{*} | 99.5 | product of experiment No. 13 was directly used, hydrogenated at 5 bar and ambient temperature, extracted with dichloromethane |
| 25 | 2.35 | 536.49 | - | - | 421.0 | 53.1^{*} | 99.8 | product of experiment No. 13 was directly used, hydrogenated at ambient pressure and temperature, extracted with dichloromethane |
| 26 | 2.35 | 628.46 | - | - | 370.0 | 46.7^{*} | 99.7 | product of experiment No. 15 was directly used, hydrogenated at ambient pressure and temperature, extracted with dichloromethane |
| 27 | 2.35 | 523.71 | - | - | 430.0 | 54.2^{*} | 99.0 | product of experiment No. 16 was directly used, hydrogenated at ambient pressure and temperature, extracted with dichloromethane |
| 28 | 2.35 | 499.91 | - | - | 345 | 43.5^{*} | 99.9 | product No. of experiment No. 17 was directly used, hydrogenated at ambient pressure and temperature, extracted with ethyl acetate |
| 29 | 2.35 | 499.91 | - | - | 366.0 | 46.2^{*} | 99.9 | product of experiment No. 18 was directly used, hydrogenated at ambient pressure and temperature, extracted with ethyl acetate |

| | | | | | | | | |
|---|---|---|---|---|---|---|---|---|
| : yield calculated starting from diketene-acetone adduct as a one-pot reaction | | | | | | | | |

¹H-NMR (400,50 MHz, DMSO-d₆): 11.23 (s, 1H, NH), 7.17 (t, 1H, CONH), 3.30-3.19 (m, 2H, CH2), 2.50-2.44 (m, 6H, 3 x CH2), 2.28 (s, 3H, ar-CH3), 2.26 (s, 3H, ar-CH3), 1.49 (s, 9H, tert-butyl), 0.94 (t, 6H, J = 7.2 Hz, 2 x CH3)
¹³C-NMR: (100,7 MHz, DMSO-d₆): 165.4, 161.0, 133.7, 125.8, 119.5, 118.4, 80.0, 52.1, 47.0, 37.4, 28.6, 12.7, 12.4, 11.8

### Example 4: Synthesis of 2,4-Dimethyl-1H-pyrrole-3-carboxylic acid (2-diethylaminoethyl)-amide (compound of the formula V)

3,5-Dimethyl-1H-pyrrole-2,4-dicarboxylic acid 2-tert-butyl ester 4-(2-diethylamino-ethyl)-amide was subjected to saponification and decarboxylation with 1 M H₂SO₄.

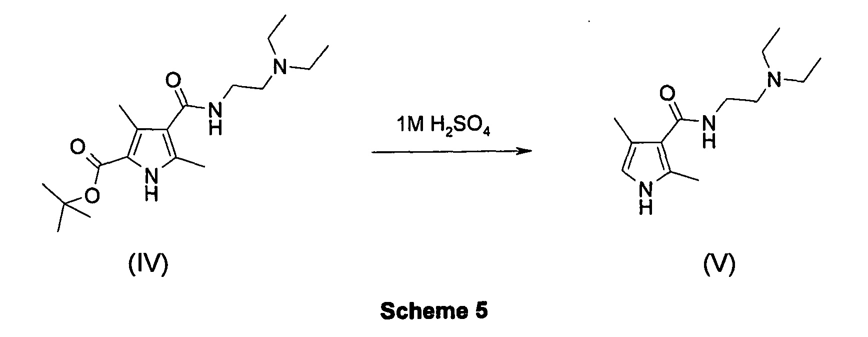

### Experimental procedure

The first batch was performed exactly as described in US 2006/0009510 A1. 3,5-Dimethyl-1 H-pyrrole-2,4-dicarboxylic acid 2-tert-butyl ester 4-(2-diethylamino-ethyl)-amide (1 equiv.) was added to a flask and 1M H₂SO₄ in methanol and water (3:1) (15 equiv. H₂SO₄) was added drop wise. The mixture was heated to 65°C for 3.5 hours. After cooling to 0-5°C, the mixture was diluted with water and basified to pH>11 with 32% aqueous sodium hydroxide. Some salts formed and were filtered off, the filtrate was extracted with dichloromethane. The organic phase was washed with water and brine, dried over sodium sulphate and evaporated to obtain a light brown oil which was used without further purification.

Alternatively, the excess of sulphuric acid, and indirectly, the amount of solvent, was decreased to 5 equivalents H₂SO₄, still yielding more than 80 % of the desired crude material.

In another experiment, the methanol was omitted and the reaction was performed with 5 equivalents and 3 equivalents of 1 M aqueous sulphuric acid respectively. These reactions yielded, after basification and extraction with ethyl acetate, in 80 % - 100 % of the alpha-free pyrrole which was again used as obtained.

A batch, where 2M sulphuric acid was used resulted in large amounts of by-products.

Another three batches with scales up to 1.5 mole were performed using a protocol with only 3 equivalents of aqueous sulphuric acid and extraction with ethyl acetate. The yields were in the range of 80 - 100 % and the crude oily products were used without further purification.

**Table 4: Overview of synthesis of 2,4-Dimethyl-1H-pyrrole-3-carboxylic acid (2-diethylaminoethyl)-amide**

| Starting material (IV) | | Product (V) | | | |
|---|---|---|---|---|---|
| [mol] | [g] | [g] | Yield (%) | Purity (%) | Remarks and observation |
| 0.006 | 2.02 | 1.8 | 126.4 | 96.0 | reaction in 1M H2SO4 (15 equiv.) in methanol, extraction with dichloromethane |
| | | | | | raw material, used without further purification |
| 0.06 | 20.25 | 12.4 | 88.4 | 96.0 | reaction in 1 M H2SO4 (7.5 equiv.) in methanol, extraction with dichloromethane |
| | | | | | raw material, used without further purification |
| 0.12 | 40.50 | 22.8 | 81.3 | 98.8 | reaction in 1 M H2SO4 (7.5 equiv.) in methanol, extraction with dichloromethane |
| | | | | | raw material, used without further purification |
| 0.8 | 269.98 | 170.7 | 91.3 | 96.0 | reaction in 1 M H2SO4 (6 equiv.) in methanol, extraction with dichloromethane |
| | | | | | raw material, used without further purification |
| 0.8 | 269.98 | 155.6 | 83.2 | 98.0 | reaction in 1 M H2SO4 (5 equiv.) in methanol, extraction with dichloromethane |
| | | | | | raw material, used without further purification |
| 0.8 | 269.98 | 161.8 | 86.5 | 99.0 | reaction in 1 M H2SO4 (5 equiv.) in methanol, extraction with dichloromethane |
| | | | | | raw material, used without further purification |
| 0.006 | 2.00 | 1.13 | 80.3 | n.d. | reaction in 1 M aqueous H2SO4 (5 equiv.), extraction with ethyl acetate |
| | | | | | raw material, used without further purification |
| 0.06 | 20.0 | 0.00 | 0.0 | n.d. | reaction in 2M aqueous H2SO4 (5 equiv.), starting material still observable while by-product forms |
| 0.03 | 10.0 | 7.14 | 101.5 | 92.6 | reaction in 1M aqueous H2SO4 (3 equiv.), extraction with ethyl acetate |
| | | | | | raw material, used without further purification |
| 0.59 | 200.0 | 155.0 | 110.4 | n.d. | reaction in 1 M aqueous H2SO4 (3 equiv.), extraction with ethyl acetate |
| | | | | | raw material, used without further purification |
| 0.30 | 100.0 | 57.0 | 81.2 | 89.0 | reaction in 1 M aqueous H2SO4 (3 equiv.), extraction with ethyl acetate |
| | | | | | raw material, used without further purification |
| 1.48 | 500.0 | 338.0 | 96.3 | 92.0 | reaction in 1M aqueous H2SO4 (3 equiv.), extraction with ethyl acetate |
| | | | | | raw material, used without further purification |
| 1.48 | 500.0 | 333.0 | 94.9 | 91.0 | reaction in 1 M aqueous H2SO4 (3 equiv.), extraction with ethyl acetate |
| | | | | | raw material, used without further purification |

### Example 5: Synthesis of Sunitinib free base (compound of the formula VI)

In a modified procedure from US2006/0009510_A1 (example 1 [132], page 16), Sunitinib free base was synthesized in a one-pot reaction. The pyrrole (2,4-Dimethyl-1H-pyrrole-3-carboxylic acid (2-diethylamino-ethyl)-amide; compound of the formula V) was first formylated by means of the Vilsmeier reagent and then condensed with 5-Fluoro-oxindole. After filtration, slurrying in water and drying, Sunitinib free base (compound of the formula VI) was obtained as crystalline form II.

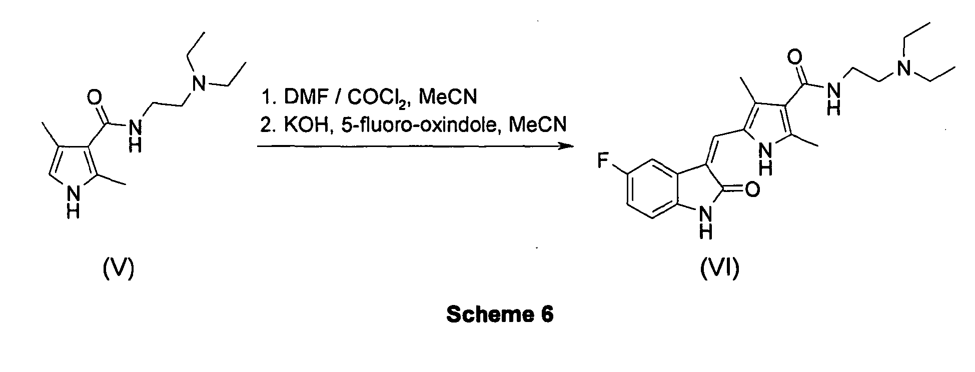

### Experimental procedure

The first five batches were carried out using solid Vilsmeier reagent (1.05-1.2 equiv.), either purchased from Merck or, in one case, synthesized by a well known procedure and isolated as white solid. This was charged to the flask and acetonitrile was added. After the drop wise addition of the pyrrole (compound of the formula V) (1 equiv.), dissolved in acetonitrile, the mixture was stirred at room temperature until all pyrrole was consumed (checked by HPLC). Then, 5-Fluoro-oxindole (0.9 equiv.) and solid potassium hydroxide (3.67 equiv.) were added and the mixture was stirred at room temperature over night. The red precipitate was filtered off and washed with acetonitrile, slurried in a large amount of water and stirred at room temperature. The now orange coloured precipitate was filtered off and washed with excess water to remove potassium salts. After drying at 50°C under vacuum until constant weight, Sunitinib free base was obtained in about 60 % yield.

An alternative procedure was carried out with *in situ* generated Vilsmeier reagent, produced by adding a solution of oxalyl chloride (1.2 equiv.) in acetonitrile to a solution of DMF (1.2 equiv.) in acetonitrile and stirring for 40 minutes before adding the pyrrole compound. The further reaction was carried out as described above, yielding again in about 60 % of Sunitinib free base with chemical purities of 96 - 99 %.

The addition of oxalyl chloride to a solution of the pyrrole compound in the presence of DMF resulted in the formation of a dark red, creamy solid. After addition of the oxindole and potassium hydroxide, Sunitinib free base was obtained, after the same work-up as described above, in only 31.5 % yield but, nevertheless, 95 % chemical purity.

**Table 5: Overview of synthesis of Sunitinib free base**

| Starting material (V) | | Product (VI) | | | |
|---|---|---|---|---|---|
| [mol] | [g] | [g] | Yield (%) | Purity (%) | Remarks and observation |
| 0.006 | 1.42 | 0.96 | 40.4 | 96.6 | Vilsmeier reagent purchased |
| 0.05 | 12.10 | 8.3 | 46.4 | 99.8 | Vilsmeier reagent purchased, crude product first stirred in ethanol then in water |
| 0.05 | 12.10 | 12.4 | 62.3 | 80.0 | Vilsmeier reagent purchased, main product fraction obtained from the acetonitrile mother liquor |
| 0.04 | 10.44 | 11.5 | 69.0 | 99.7 | Vilsmeier reagent purchased, crude product washed with only little acetonitrile before slurrying in water |
| 0.72 | 170.89 | 187.0 | 68.6 | 99.0 | Vilsmeier reagent generated and isolated before, crude product washed with only little acetonitrile before slurrying in water |
| 0.005 | 1.12 | 0.56 | 31.5 | 95.7 | Vilsmeier reagent generated *in situ* in presence of V, dark red, creamy solid formed |
| 0.03 | 7.14 | 7.53 | 68.3 | 96.9 | Vilsmeier reagent generated *in situ* before addition of V |
| 0.66 | 155.6 | 161.2 | 63.0 | 98.9 | Vilsmeier reagent generated *in situ* before addition of V |
| 0.68 | 161.8 | 172.3 | 64.7 | 95.7 | Vilsmeier reagent generated *in situ* before addition of V |
| 0.24 | 57.0 | 59.0 | 62.9 | 99.5 | Vilsmeier reagent generated *in situ* before addition of V |
| 1.18 | 280.0 | 272.0 | 59.0 | 99.4 | Vilsmeier reagent generated *in situ* before addition of V |
| 1.47 | 350.0 | 283.0 | 53.5 | 99.4 | Vilsmeier reagent generated *in situ* before addition of V |

¹H-NMR (400,50 MHz, DMSO-d₆): 13.37 (s, 1H, pyrrole-NH), 10.87 (s, 1H, indole-NH), 7.74 (dd, 1H, 3J(H,F) = 9.4 Hz, 4J = 2.2 Hz, ar-H), 7.70 (s, 1H, vinyl-H), 7.41 (t, 3J = 5.4 Hz, CONH), 6.90 (dt, 1H, 3J(H,F) = 9.6 Hz, 3J(H,H) = 8.4 Hz, 4J(H,H) = 2.2 Hz, ar-H), 6.82 (dd, 1H, 3J(H,H) = 8.6 Hz, 4J(H,F) = 4.6 Hz, ar-H), 3.26 (quart, 2H, CH2), 2.53-2.41 (m, 6H, 3 x CH2), 2.42 (s, 3H, pyrrole-CH3), 2.40 (s, 3H, pyrrole-CH3), 0.96 (t, 6H, 3J(H,H) = 7.0 Hz, 2 x CH3)
¹³C-NMR: (100,7 MHz, DMSO-d₆):170.0, 164.9, 158.7 (d, J = 234.4 Hz), 137.0, 135.0, 130.6, 127.6 (d, J = 9.6 Hz), 126.2, 125.3, 121.2, 115.1, 112.8 (d, J = 24.4 Hz), 110.5 (d, J = 8.1 Hz), 106.4 (d, J = 25.8 Hz), 52.1, 47.0, 37.4, 13.8, 12.4, 11.1
IR (υ/cm⁻¹) = 3276, 3156, 3111, 3041, 2967, 2931, 1667, 1621, 1556, 1476, 1444, 1384, 1325, 1306, 1261, 1240, 1200, 1180, 1165, 1147, 1095, 1068, 1026, 1000, 926, 920, 877, 858, 793, 776, 752, 726, 697, 667, 608

### Example 6: Synthesis of the L-(-)-malate salt of Sunitinib

Sunitinib free base (compound of the formula VI) was reacted with L-(-)-malic acid in methanol to give Sunitinib L-malate salt.

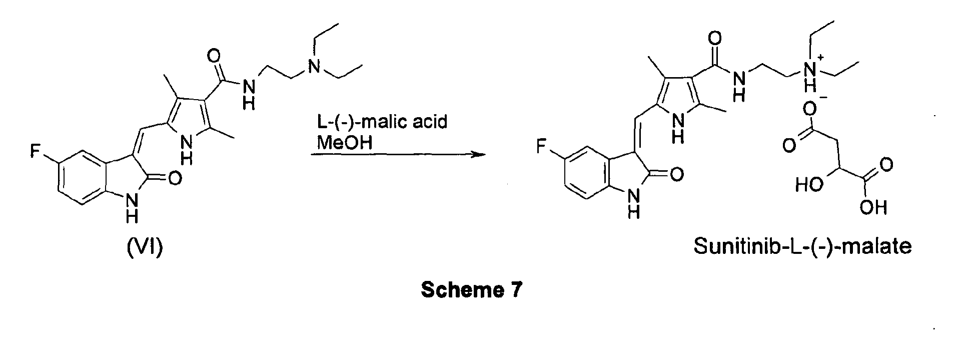

An improved procedure compared to that described in WO2001\60814 A2 was carried out for the formation of the L-(-)-malate salt: Sunitinib (compound of the formula VI) was suspended in prewarmed methanol at 50°C and L-(-)-malic acid was added. The solids dissolved gradually. When no more solid was observed, the mixture was cooled to 4-10°C with the help of an ice-bath. The crystals were filtered off and washed with methanol to obtain about 90 % of the theoretical yield of Sunitinib L-(-)-malate in excellent chemical purity of > 99%.

To get a uniform batch, the three main batches were slurried in methanol and stirred for 3.5 hours at room temperature. After filtration and washing with methanol the L-malate salt of Sunitinib was obtained in 99.2 % yield with a chemical purity of 99.4 %.

**Table 6: Overview of synthesis of Sunitinib-L-(-)-malate**

| Starting material (VI) | | Product (Sunitinib-L-(-)-malate) | | | |
|---|---|---|---|---|---|
| [mol] | [g] | [g] | Yield (%) | Purity (%) | Remarks and observation |
| 0.13 | 5.00 | 5.96 | 89.2 | 98.7 | excess methanol distilled off prior to crystallization |
| 0.13 | 5.00 | 5.32 | 83.2 | 99.4 | malic acid added to methanolic solution of Sunitinib base at 50°C, crystallization upon cooling, crystals slurried in methanol for purification |
| 0.31 | 125.0 | 155.5 | 93.8 | 99.6 | malic acid and Sunitinib heated in methanol to 40°C to get a clear solution, crystallization upon cooling |
| 0.38 | 150.0 | 178.6 | 89.8 | 99.2 | malic acid and Sunitinib heated in methanol to 40°C, crystallization upon cooling |
| 0.45 | 180.0 | 216.6 | 90.8 | 99.4 | malic acid added to methanolic solution of Sunitinib base at 50°C, crystallization upon cooling, crystals slurried in methanol for purification |

### Example 7: Synthesis of Sunitinib free base, Form III

The recrystallization of Sunitinib free base form II into form III was carried out in refluxing acetone. Three different crystallization conditions were tried out and the resulting crystalline form of Sunitinib was examined. In preliminary experiments, the form II free base was dissolved in refluxing acetone, undissolved solids were filtered off and the clear solution was seeded with crystalline form III. The solvent was reduced - once at room temperature under vacuum and once at 63°C under ambient pressure - and after two hours the precipitate was filtered off, washed with acetone and dried under vacuum. In the batch which was reduced at room temperature crystalline form III was obtained in 70 % yield, in the one reduced at 63°C, form II was recovered.

Because of the large volumes required for complete dissolution of Sunitinib form II, an alternative protocol was tried out using a Soxhlet apparatus and extracting the Sunitinib with acetone. Therefore, a saturated solution of Sunitinib form II in acetone was formed and seeded with some crystals of form III. The Soxhlet tube was filled with more Sunitinib form II which was extracted until the tube was empty. In a first small batch, crystalline form III was obtained in high yield. A second, larger batch afforded a large amount of form I as well as some form III which could be separated due to their different solubility. Form I was afterwards recrystallized to form III from large volumes of acetone, yielding altogether 94.5% of form III.

Alternatively Sunitinib form II was refluxed in acetone to get a saturated solution. Undissolved solids were filtered off while hot and the clear solution was seeded with form III. The solvent was partially evaporated under vacuum using a water bath tempered at 30-40°C and the residual solution was cooled to room temperature. The precipitate was filtered off, washed with acetone and dried under vacuum.

Using this protocol, 253,7 g of Sunitinib free base form III was synthesized. The yields varied between 68 % und 90 %, chemical purities were constantly about 99,9 %.

## Claims

1. Process for the preparation of a compound of the formula I 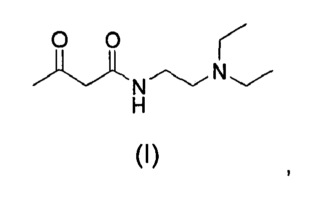 or a salt thereof
which process comprises the step of reacting a compound of the formula II 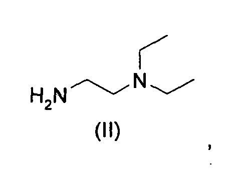 or a salt thereof
with 2,2,6-trimethyl-1,3-dioxin-4-one.

2. Process according to claim 1, which process further comprises the step of reacting the compound of formula I 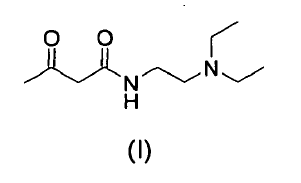 with a compound of formula III 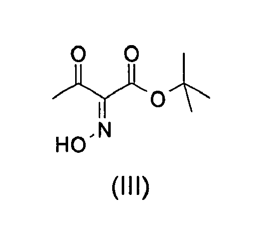 to obtain a compound of the formula IV 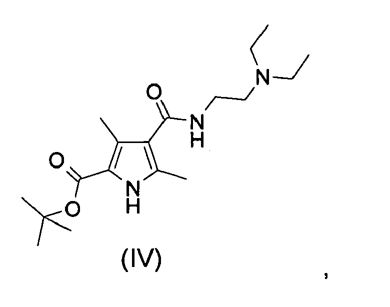 or a salt thereof.

3. Process according to claim 2, which process further comprises the step of reacting the compound of formula IV 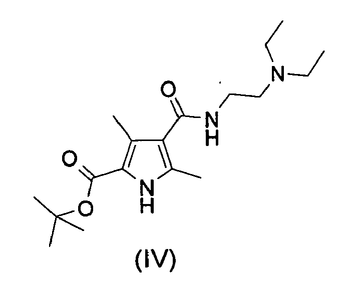 or a salt thereof
to obtain a compound of the formula V 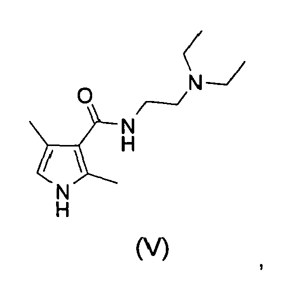 or a salt thereof.

4. Process according to claim 3 wherein the reaction is conducted without application of an organic solvent.

5. Process according to claim 3 or 4, which process further comprises the step of reacting the compound of the formula V 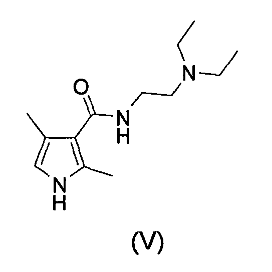 or a salt thereof,
to obtain a compound oft he formula VI 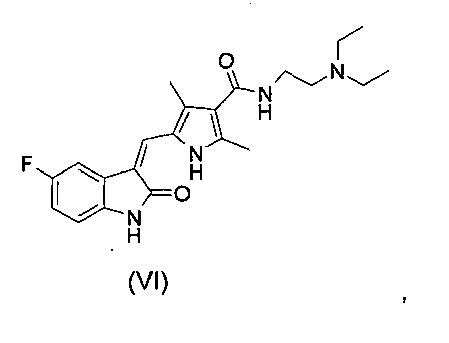 or a salt thereof.

6. Process according to claim 5, wherein the compound of formula VI is obtained as the crystalline free base in polymorphic form II.

7. Process according to claim 5 or 6, which process further comprises the step of recrystallization of the compound of the formula VI 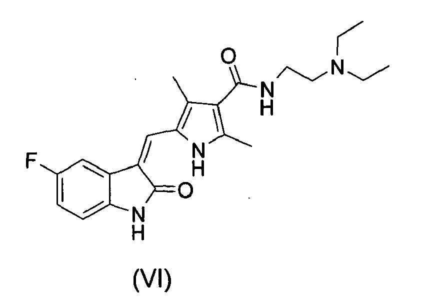 to obtain the crystalline compound of formula VI in polymorphic form III.

8. Process according to any one of claims 5 to 7, which process further comprises the step of reacting the compound of the formula VI 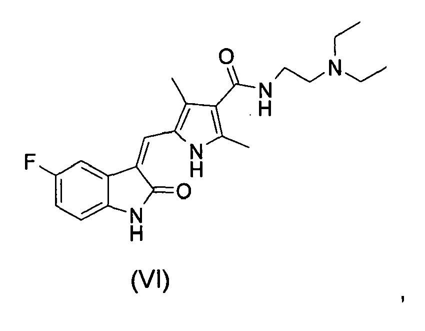 or a salt thereof,
to obtain the L-(-)-malate salt of the compound of formula VI.
